(19) Europäisches Patentamt
European Patent Office
Office européen des brevets

(11) Veröffentlichungsnummer: **0 132 714**
**B1**

(12) # EUROPÄISCHE PATENTSCHRIFT

(45) Veröffentlichungstag der Patentschrift: **28.01.87**

(21) Anmeldenummer: **84108204.3**

(22) Anmeldetag: **12.07.84**

(51) Int. Cl.⁴: **C 07 D 215/04**, C 07 D 215/48, C 07 D 215/18, C 07 D 215/24, C 07 D 215/38, C 07 D 215/40, C 07 D 513/04, C 07 D 471/04, C 07 D 491/048, C 07 D 495/04, C 07 D 498/04

(54) **Verfahren zur Herstellung von Chinolinen.**

(30) Priorität: **21.07.83 DE 3326255**

(43) Veröffentlichungstag der Anmeldung: **13.02.85 Patentblatt 85/7**

(45) Bekanntmachung des Hinweises auf die Patenterteilung: **28.01.87 Patentblatt 87/5**

(84) Benannte Vertragsstaaten: **BE CH DE FR GB LI**

(56) Entgegenhaltungen: **CH - A - 459 204**

(73) Patentinhaber: **BASF Aktiengesellschaft, Carl-Bosch-Strasse 38, D-6700 Ludwigshafen (DE)**

(72) Erfinder: **Dockner, Toni, Dr., Grossgasse 6, D-6701 Meckenheim (DE)**
Erfinder: **Hagen, Helmut, Dr., Max-Slevogt-Strasse 17e, D-6710 Frankenthal (DE)**
Erfinder: **Kohler, Rolf-Dieter, Dr., Amselweg 3, D-6803 Edingen-Neckarhausen (DE)**
Erfinder: **Markert, Juergen, Dr., Am Speyerweg 26, D-6704 Mutterstadt (DE)**

EP 0 132 714 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Chinolinen durch Umsetzung von aromatischen Aminen mit Acroleinen oder seinen Acetalen in Gegenwart von organischen Lösungsmitteln und Säuren als Katalysatoren.

Es ist aus den Chem. Ber. 12 (1879), Seite 453, bekannt, Allylanilin mit Bleioxid bei hoher Temperatur zu Chinolin umzusetzen. Das Reaktionsgemisch wird mit Schwefelsäure versetzt, filtriert, das Filtrat mit Ether ausgeschüttelt, mit einer Lösung von chromsauren Kali gekocht, alkalisch gemacht und mit Wasserdampf destilliert, um das Chinolin zu isolieren. Bei der allgemein verwendeten Methode, der Skraupschen Chinolinsynthese, wird Anilin mit Glycerin in konz. Schwefelsäure in Gegenwart von Nitrobenzol umgesetzt (Chem. Ber. 13, 2086 bis 2087 (1880). Bei der bekannten Variante nach Doebner und von Miller wird Anilin mit Paraldehyd in Gegenwart von Nitrobenzol und von konz. Schwefelsäure zu 2-Methylchinolin cyclisiert (Chem. Ber. 14, 2812 bis 2817 (1881). Man verwendet die Schwefelsäure in einem Molverhältnis von mehr als 2 Mol Säure, bezogen auf Anilin. Ebenfalls ist die Umsetzung von Anilin mit Acetylaceton unter leichtem Erhitzen bekannt; das Reaktionsgemisch wird in konzentrierter Schwefelsäure aufgelöst, erhitzt, das Gemisch in kaltes Wasser eingegeben und überschüssiges Ammoniak zugefügt. Dann wird die organische Schicht in Ether aufgenommen und das Gemisch fraktioniert destilliert. In entsprechender Weise wurde das 2,3,4-Trimethylchinolin hergestellt (Bull. Soc. Chim. de Paris [2], 49 (1888), Seiten 89 bis 92).

Zwar lehrt Rodd, Chemistry of Carbon Compounds, Band IV A (N.Y. 1957), Seiten 586 bis 594, dass unter den Bedingungen der Skraupschen Synthese, nämlich mit Glycerin als Ausgangsstoff in Gegenwart von konzentrierter Schwefelsäure und Oxidationsmitteln, Acrolein intermediär entsteht, er weist aber darauf hin (loc. cit. S. 588), dass Chinolin aus Anilin und Acrolein nicht oder nur in schlechten Ausbeuten erhalten wird, also Acrolein in situ erzeugt werden muss. Nur gewisse Nitroaniline sollen sich mit Acrolein in Gegenwart von Phosphorsäure umsetzen. Weiterhin werden bei diesen Umsetzungen stets Oxidationsmittel, z.B. Nitrobenzol oder Arsensäure, und als Säure konzentrierte Mineralsäuren, z.B. auch konzentrierte Salzsäure, verwendet. Rodd erläutert, dass bei der Döbner-von Miller-Synthese ebenfalls Mineralsäure, im allgemeinen Salzsäure, aber kein Oxidationsmittel verwendet wird. Als Ausgangsstoffe werden gesättigte Aldehyde wie Acetaldehyd verwendet.

In der CH-PS 459 204 wird beschrieben, dass Methacroleindiacetat mit Nitroanilin in Gegenwart von Arsensäure zu 3-Methyl-8-nitrochinolin umgesetzt werden kann.

Die Verwendung von Arsensäure als Oxidationsmittel bei der Skraup'schen Chinolinsynthese geht auch aus Quinolines (Teil I) von G. Jones, Wiley 1977, Seiten 100 bis 117 und 291, hervor. Diese Schrift lehrt weiterhin, dass die Umsetzung in der Regel in Gegenwart von Schwefelsäure durchgeführt wird, dass jedoch durch eine Kombination von Schwefelsäure mit Essigsäure eine Ausbeutesteigerung erzielt werden konnte (s. Seite 116). Werden Acroleine als Ausgangsstoffe verwendet, so kann deren Polymerisation herabgesetzt werden, wenn man sie in Form ihrer Derivate wie Acetale oder Acetate einsetzt. Bei der Herstellung von 5-Hydroxy-8-Methylchinolin (Verbindung 85, Seite 111) wird, wie aus der Originalliteratur H.J. Tauber u. S. Benz in Chem. Ber. 100 (1967), S. 2918 bis 2929, insbesondere S. 2923 hervorgeht, 2-Amino-4-hydroxytoluol in Methanol mit β-Äthoxyacrolein-diäthylacetal versetzt, das Lösungsmittel nach 24 h verdampft und durch Eisessig ersetzt, das Reaktionsgemisch erhitzt, das Lösungsmittel erneut entfernt und der letzte Umsetzungsschritt in verdünnter Schwefelsäure, der konzentrierte Schwefelsäure zugesetzt wird, vorgenommen. Trotz dieses aufwendigen Verfahrens lässt sich die Bildung harziger Nebenprodukte nicht vermeiden, so dass das Endprodukt nach dessen Reinigung nur in einer Ausbeute von 15% anfällt.

Es wurde nun gefunden, dass man Chinoline der Formel

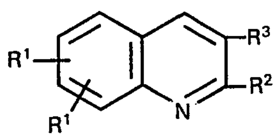

I,

worin die einzelnen Reste $R^1$, $R^2$ und $R^3$ gleich oder verschieden sein können und jeweils ein Wasserstoffatom oder einen aliphatischen Rest bedeuten, die Reste $R^1$ auch jeweils ein Halogenatom, eine Alkoxygruppe, eine Aminogruppe, eine Monoalkyl- oder eine Dialkylaminogruppe, oder zusammen mit zwei benachbarten Kohlenstoffatomen einen Isoxazolrest, Oxazolrest, Thioazolrest, Isothiazolrest, Furanrest, Thiophenrest, Imidazolrest oder Pyrazolrest bezeichnen, durch Umsetzung von aromatischen Aminen der Formel

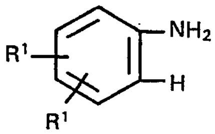

II,

worin $R^1$ die vorgenannte Bedeutung besitzt, mit Acroleinen oder seinen Acetalen der Formel

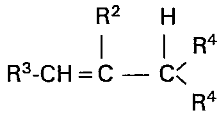

III,

worin die einzelnen Reste $R^4$ gleich oder verschieden sein können und jeweils für eine Alkoxygruppe oder zusammen für ein Sauerstoffatom stehen, in Gegenwart von Säure und organischen Lösungsmitteln vorteilhaft erhält, wenn man als Säure organische Säuren verwendet und die Umsetzung in Gegenwart von unter den Reaktionsbedingungen inerten, nitrogruppenfreien, organischen Lösungsmitteln durchgeführt.

Die Umsetzung kann für den Fall der Verwendung von 3-Chlor-o-toluidin und Acrolein durch die folgenden Formeln wiedergegeben werden:

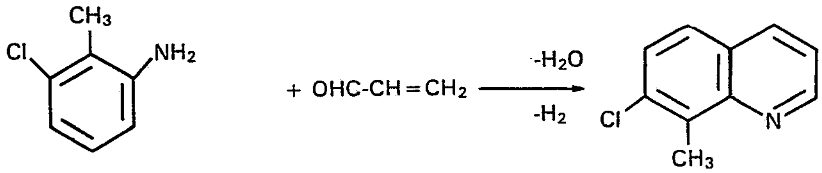


Im Hinblick auf die bekannten Verfahren liefert das Verfahren nach der Erfindung auf einfacherem und wirtschaftlicherem Wege Chinoline, insbesondere zahlreiche, bisher nur schwierig herstellbare, substituierte Chinoline, in besserer Ausbeute und Reinheit. Insbesondere sind die Anthranilsäuren als Katalysatoren im Hinblick auf die Ausbeuten vorteilhaft. Zusätzliche Oxidationsmittel werden nicht benötigt. Ebenfalls werden kein Nitrobenzol und keine Mineralsäuren, die während der Reaktion die Bildung von Zersetzungsprodukten und harzigen Rückständen fördern, verwendet. Anstelle hoher Mengen Mineralsäure genügen schon katalytische Mengen an organischen Säuren, was nicht nur wirtschaftliche Vorteile, sondern auch Vorteile bezüglich einfacherem Betrieb (Abführung der Neutralisationswärme, Einsparung von Neutralisationsmitteln und Operationen) und Umweltschutz (Vermeidung hoher Salzmengen, geringere Abwasserprobleme) mit sich bringt. Alle diese vorteilhaften Ergebnisse sind im Hinblick auf den Stand der Technik überraschend. Es war mit Bezug auf die vorgenannten Veröffentlichungen nicht zu erwarten, dass eine Umsetzung in Abwesenheit von Oxidationsmitteln und Mineralsäuren und unter Verwendung katalytischer Mengen an organischen Säuren zu wesentlichen und noch dazu besseren Ausbeuten an Chinolinen führt. Insbesondere war es im Hinblick auf Rodd (loc. cit.) überraschend, dass Acroleine auch als Ausgangsstoffe mit guten Ergebnissen verwendet werden können. Es war nicht zu erwarten, dass sogar Ausgangsstoffe II, die durch die Carboxylgruppe substiutuiert sind, gleichzeitig als Katalysator dienen können.

Die Ausgangsstoffe II und III können in stöchiometrischer Menge oder im Überschuss jeder Komponente zur anderen, vorteilhaft in einem Verhältnis von 0,8 bis 4, insbesondere 1 bis 2 Mol Ausgangsstoff III je Mol Ausgangsstoff II, umgesetzt werden. Verwendet man Ausgangsstoffe II, die 2 oder 2 Aminogruppen im Molekül tragen, so wird in der Regel ein Molverhältnis von höchstens 1,5, zweckmässig 0,8 bis 1,5, vorteilhaft 1 bis 1,5, insbesondere 1,1 bis 1,3 Mol Ausgangsstoff III je Mol Ausgangsstoff II gewählt. Verwendet man ein Verhältnis von mehr als 1,5 Mol Ausgangsstoff III, so bilden sich in mit grösseren Mengen an Ausgangsstoff III steigenden Mengen Phenanthroline bzw. entsprechende Triazaverbindungen mit 4 Ringen im Molekül. Bevorzugte Ausgangsstoffe II und III und dementsprechend bevorzugt Endstoffe I sind solche, in deren Formeln die einzelnen Reste $R^1$, $R^2$ und $R^3$ gleich oder verschieden sein können und jeweils ein Wasserstoffatom, eine Carboxylgruppe oder einen Alkylrest mit 1 bis 8, insbesondere 1 bis 4 Kohlenstoffatomen bedeuten, die Reste $R^1$ auch jeweils ein Fluoratom, Chloratom oder Bromatom, eine Aminogruppe, eine Alkoxygruppe, eine Monoalkyl- oder Dialkylaminogruppe mit jeweils 1 bis 8, insbesondere 1 bis 4 Kohlenstoffatomen je Alkylgruppe, oder zusammen mit zwei benachbarten Kohlenstoffatomen einen Isoxazolrest, Oxazolrest, Thiazolrest, Isothiazolrest, Furanrest, Thiophenrest, Pyrrolrest, Imidazolrest oder Pyrazolrest bezeichnen, die einzelnen Reste $R^4$ gleich oder verschieden sein können und jeweils für eine Alkoxygruppe mit 1 bis 4 Kohlenstoffatomen oder zusammen für ein Sauerstoffatom stehen. Die vorgenannten Reste können noch durch unter den Reaktionsbedingungen inerte Gruppen oder Atome, z.B. Chloratome, Bromatome, Alkylgruppen, Alkoxygruppen mit jeweils 1 bis 4 Kohlenstoffatomen, substituiert sein.

Beispielsweise sind folgende Ausgangsstoffe II geeignet: Anilin, o-Methyl-, o-Ethyl-, o-Propyl-, o-Isopropyl-, o-Butyl-, o-Isobutyl-, o-sek.-Butyl-, o-tert.-Butyl-, o-Methoxy-, o-Ethoxy-, p-Propoxy-, o-Isopropoxy-, o-Butoxy-, o-Isobutoxy-, o-sek.-Butoxy-, o-tert.-Butoxy-, o-Dimethylamino-, o-Diethylamino-, o-Dipropylamino-, o-Dibutylamino-, o-Diisobutylamino-, o-Di-sek.-butylamino-, o-Di-tert.-butylamino-, o-N-Methyl-N-ethylamino-, o-Chlor-, o-Brom-, o-Carboxy-anilin; anstelle der vorgenannten Dialkylaminogruppen mit entsprechenden Monoalkylaminogruppen oder der unsubstituierten Aminogruppe substituierte Aniline; durch vorgenannte Gruppen meta-substituierte oder parasubstituierte Aniline; in 2,4-, 2,3-, 2,5-, 3,4- oder 3,5-Stellung durch vorgenante Gruppen gleich oder verschieden zweifach substituierte Aniline; in 4-Stellung, 5-Stellung, 6-Stellung oder 7-Stellung durch die Aminogruppe substituiertes Benzofuran, Thionaphthen, Indol, Benzoxazol, Benzpyrazol, Benzimidazol, Benzisoxazol, Benzthiazol, Benzisothiazol.

Folgende Ausgangsstoffe III können beispielsweise verwendet werden; Acrolein; in 2-Stellung und/oder 3-Stellung durch gleiche oder unterschiedliche Methyl-, Ethyl-, Propyl-, Isopropyl-, Butyl-, Isobutyl-, sek.-Butyl-, tert.-Butyl-gruppen substituierte Acroleine; entsprechende Dimethyl-, Diethyl-, Dipropyl-, Diisopropyl-, Dibutyl-, Diisobutyl-, Di-sek.-butyl-, Di-tert.-butylacetale vorgenannter Acroleine.

Die Umsetzung wird im allgemeinen bei einer Temperatur von 40 bis 200, vorzugsweise von 100 bis

180°C, drucklos oder unter Druck, kontinuierlich oder diskontinuierlich durchgeführt. Man verwendet unter den Reaktionsbedingungen inerte, organische, nitrogruppenfreie Lösungsmittel. Als Lösungsmittel kommen z.B. in Frage: aromatische Kohlenwasserstoffe, z.B. Toluol, 1,3,5-Trimethylbenzol, Ethylbenzol, o-, m-, p-Xylol, o-, m-, p-Diethylbenzol, Isopropylbenzol, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol; Sulfoxide wie Dimethylsulfoxid, Diethylsulfoxid, Dimethylsulfon, Diethylsulfon, Methylethylsulfon, Tetramethylensulfon; Alkanole und Cycloalkanole wie Ethanol, Methanol n-Butanol, Isobutanol, tert.-Butanol, Glykol, 3-Methoxypropanol, sek.-Butanol, n-Propanol, Isopropanol, Cyclohexanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, Diacetonalkohol; Ether, z.B. Ethylpropylether, Methyl-tert.-butylether, n-Butylethylether, Di-n-butylether, Diisobutylether, Diisopropylether, Diethylether, Diethylenglykoldimethylether, Diethylenglykoldiethylether, Tetrahydrofuran, Dioxan, Thioanisol; und entsprechende Gemische. Zweckmässig verwendet man das Lösungsmittel in einer Menge von 400 bis 10 000 Gewichtsprozent, vorzugsweise von 500 bis 2000 Gewichtsprozent, bezogen auf Ausgangsstoff II.

Die Umsetzung wird in Gegenwart von organischer Säure, in der Regel in katalytischen Mengen, vorteilhaft mit einer Menge von 0,001 bis 0,4, insbesondere von 0,005 bis 0,05 Äquivalenten Säure, bezogen auf 1 Mol Ausgangsstoff II, durchgeführt. Anstelle einbasischer Säuren können auch äquivalente Mengen mehrbasischer Säuren zur Anwendung gelangen. Beispielsweise sind folgende Säuren geeignet: Sulfonsäuren wie Benzol- und p-Toluolsulfonsäure, Dodecylbenzolsulfonsäure; aliphatische Carbonsäuren wie Oxalsäure, Ameisensäure, Essigsäure, Propionsäure, Capronsäure, Caprinsäure, 3,5,5-Trimethylhexansäure, Laurinsäure, Palmitinsäure, Stearinsäure, 2-Ethylhexancarbonsäure, α-Ethylbuttersäure, 2-Methylbutansäure, Glykolsäure, Milchsäure, Brenztraubensäure, Weinsäure, Caprylsäure, Trimethylessigsäure, Bernsteinsäure, Isovaleriansäure, Valeriansäure, Glutarsäure, Adipinsäure; cycloaliphatischen, araliphatischen, aromatischen Carbonsäuren wie Benzoesäure, 2,3-, 2,4-, 2,5-, 2,6-Dimethylbenzoesäure, Mellithsäure, Phenylpropionsäure, o-, m- bzw. p-Chlorbenzoesäure, Cyclohexancarbonsäure, Cyclopentancarbonsäure, Phenylessigsäure, α-bzw. β-Naphthoesäure, Phthalsäure, o-, m- bzw. p-Toluylsäure, Isophthalsäure, Terephthalsäure oder entsprechende Gemische. Bevorzugt sind Toluolsulfonsäuren, Pikrinsäure, Benzolsulfonsäure, 6-Fluoranthranilsäure, 6-Chloranthranilsäure, Dodecylbenzolsulfonsäure. Enthalten die Ausgangsstoffe, insbesondere Ausgangsstoff II, Carboxylgruppen, sind also selbst Säuren, so wird in einer bevorzugten Ausführungsform keine zusätzlich Säure, sondern der Ausgangsstoff gleichzeitig als Katalysator verwendet; in solchen Fällen ist ein Molverhältnis von 0,8 bis 4, vorteilhaft 1 bis 2 Mol Ausgangsstoff III je Mol Ausgangsstoff II zweckmässig.

Die Reaktion kann wie folgt durchgeführt werden: Ein Gemisch von Ausgangsstoff II und III, Säure und Lösungsmittel wird während 0,5 bis 20, vorteilhaft 0,5 bis 6 Stunden bei der Reaktionstemperatur gehalten. Dann wird der Endstoff aus dem Gemisch in üblicher Weise, z.B. durch fraktionierte Destillation, agbetrennt. Zwar kann Sauerstoff bzw. Luft während der Reaktion anwesend sein, in der Regel werden aber zusätzliche Oxidationsmittel wie Bleiperoxid, Nitrobenzol, Arsensäure, nicht zugesetzt. Als Nebenprodukte bei der Reaktion können die entsprechenden Tetrahydrochinoline, im allgemeinen in Ausbeuten bis zu 27, meist von ca. 15% der Theorie, gebildet werden. Sie werden zweckmässig bei der Aufarbeitung der Endstoffe, z.B. bei der Destillation, abgetrennt und mit einem Oxidationsmittel (Nitroverbindungen Sauerstoff und Katalysatoren; Schwefel, vorteilhaft Schwefelsäure) zu den Endstoffen I oxidiert.

Die nach dem Verfahren der Erfindung herstellbaren Chinoline sind wertvolle Ausgangsstoffe für die Herstellung von Farbstoffen, Pflanzenschutzmittel und Pharmazeutika. Bezüglich der Verwendung wird auf vorgenannte Veröffentlichungen und Ullmanns Encyklopädie der technischen Chemie (4. Aufl.) Band 8, Seiten 36 und 37, Band 9, Seiten 311 und 312, Band 10, Seite 52, verwiesen.

*Beispiel 1*

70 g 3-Chlor-o-toluidin und 1 g p-Toluolsulfonsäure wurden in 700 ml Dichlorbenzol auf 150°C erhitzt. Bei dieser Temperatur wurden innerhalb 1 Stunde 28 g Acrolein (suspendiert in 200 ml Dichlorbenzol) in die Lösung eingetragen, das Dichlorbenzol abdestilliert und der Rückstand über eine Kolonne destilliert. Es wurden 68 g 7-Chlor-8-methylchinolin vom Schmelzpunkt 42 bis 43°C erhalten. Die Ausbeute entsprach 77% der der Theorie.

*Beispiel 2*

21,4 g p-Toluidin und 1 g p-Toluolsulfonsäure wurden in 400 ml Xylol auf 120°C erhitzt. Es wurden bei dieser Temperatur 28 g 2-Methacrolein während 30 Minuten in die Lösung eingetragen. Das Gemisch wurde bei dieser Temperatur 1 Stunde nachgerührt. Nach dem Abdestillieren von Xylol wurde der Rückstand über eine Kolonne destilliert. Es wurden 23 g 3,6-Dimethylchinolin vom Schmelzpunkt 56°C (aus Xylol) erhalten. Die Ausbeute entsprach 73% der Theorie.

*Beispiel 3*

31 g 6-Fluoranthranilsäure wurden in 200 ml Dichlorbenzol auf 70°C erhitzt. Bei dieser Temperatur wurden 25 g 2-Ethylacrolein während 20 Minuten in die Lösung eingetragen. Das Gemisch wurde 20 Minuten bei dieser Temperatur nachgerührt. Die Lösung wurde abgekühlt, der ausgefallene Feststoff abgesaugt und aus Toluol umkristallisiert. Es wurden 30 g 3-Ethyl-7-fluor-chinolin-8-carbonsäure vom Schmelzpunkt 95°C erhalten (69% der Theorie).

*Beispiel 4*

13 g 2-Chloranilin und 0,5 g p-Toluolsulfonsäure wurden in 200 ml Chlorbenzol auf 120°C erhitzt. Es wurden bei 120°C 14 g Crotonaldehyd während 45 Minuten in die Lösung getropft. Das Gemisch wurde 1 Stunde bei 120°C gerührt, das Lösungsmittel abdestilliert und der Rückstand über eine Kolonne fraktioniert. Es wurden 10 g 8-Chlor-2-methylchinolin vom Schmelzpunkt 64°C erhalten. Die Ausbeute betrug 57% der Theorie.

*Beispiel 5 bis 42*

Die in Tabelle 1 aufgeführten Umsetzungen wurden mit einer Menge von 0,1 Mol Ausgangsstoff II analog Beispiel 1 durchgeführt, wobei das Molverhältnis der Ausgangsstoffe II und III beibehalten wurde. Die Ausgangsstoffe und die Ergebnisse der Umsetzung werden in der Tabelle I aufgeführt. In der Fällen, in denen der Ausgangsstoff II eine Carboxylgruppe enthielt, wurde keine p-Toluolsulfonsäure zugesetzt und das Molverhältnis betrug 0,5 Mol Ausgangsstoff III je Mol Ausgangsstoff II. Die Ausbeute in den Beispielen 41 und 42 bezieht sich auf das entsprechende Aminochinolin, das mit einem Molverhältnis von 1,2 Mol Ausgangsstoff III je Mol Ausgangsstoff II hergestellt wurde.

TABELLE 1

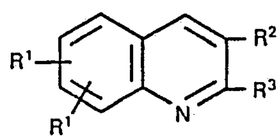

| Nr. | $R^1$ | $R^1$ | $R^2$ | $R^3$ | Fp in °C | Kp in °C (mbar) | Ausbeute % der Theorie |
|---|---|---|---|---|---|---|---|
| 5 | 8-$CH_3$ | H | H | H | | 128 (15) | 74 |
| 6 | 8-COOH | 7-Cl | H | $CH_3$ | 168 | | 57 |
| 7 | 8-COOH | 7-F | H | $CH_3$ | 180 | | 55 |
| 8 | 8-COOH | 7-Cl | $CH_3$ | H | 244 | | 72 |
| 9 | 8-COOH | 7-F | $CH_3$ | H | 170 | | 67 |
| 10 | 8-COOH | 7-Cl | $C_2H_5$ | H | 200 | | 69 |
| 11 | 8-COOH | 7-F | $C_3H_7$ | H | 124 | | 63 |
| 12 | 8-COOH | 7-Cl | $C_3H_7$ | H | 200 | | 64 |
| 13 | 8-COOH | 6-Cl | H | H | 225 | | 68 |
| 14 | 8-COOH | H | $CH_3$ | H | 161 | | 64 |
| 15 | 8-$CH_3$ | 7-$CH_3$ | $CH_3$ | H | 60 | | 75 |
| 16 | 8-Cl | 7-Cl | H | H | 90 | | 77 |
| 17 | 8-Cl | H | H | H | | 178 (16) | 75 |
| 18 | 8-Cl | H | $CH_3$ | H | | 117-118 (1) | 72 |
| 19 | 8-$OCH_3$ | H | H | H | | 119 (1) | 75 |
| 20 | 5,6-(-CH=N-S-) | | H | H | 113 | | 72 |
| 21 | 5,6-(-CH=N-S) | | H | $CH_3$ | 126 | | 74 |
| 22 | 6-$OCH_3$ | H | $CH_3$ | H | 75 | | 74 |
| 23 | 6-$N(CH_3)_2$ | H | $CH_3$ | H | 39 | | 70 |
| 24 | 8-Cl | 6-Cl | H | H | 104 | | 72 |
| 25 | 6-Cl | H | $CH_3$ | H | 81 | | 70 |
| 26 | 6-$OCH_3$ | H | H | H | | 165 (16) | 75 |
| 27 | 6-Br | H | $CH_3$ | H | 103 | | 74 |
| 28 | 6-$OCH_3$ | H | H | $CH_3$ | 67-68 | | 72 |
| 29 | 8-$CH_3$ | 5-$CH_3$ | H | H | | 105 (1) | 72 |
| 30 | 8-Cl | 7-Cl | H | H | 94 | | 76 |
| 31 | 6-F | H | H | H | | 108-110 (16) | 74 |
| 32 | H | H | $CH_3$ | H | | 80 (2) | 72 |
| 33 | H | H | H | $CH_3$ | | 128 (15) | 59 |
| 34 | 8-COOH | 5-Cl | H | H | 210 | | 66 |
| 35 | 8-COOH | 7-Cl | H | H | 244 | | 67 |
| 36 | 8-$CH_3$ | 6-$CH_3$ | H | H | | 135 (16) | 72 |
| 37 | 8-$CH_3$ | 7-Cl | $C_2H_5$ | H | | 125 (2) | 74 |
| 38 | 8-Br | H | H | H | | 160 (15) | 74 |
| 39 | 8-Cl | 7-Cl | $CH_3$ | H | 107 | | 72 |
| 40 | 8-COOH | 7-Cl | $C_5H_{11}$ | H | 133 | | 64 |
| 41 | 8-$NH_2$ | H | $CH_3$ | H | 86 | | 74 |
| 42 | 6-$NH_2$ | H | H | H | 113 | | 72 |

*Beispiel 43 bis 55*

Analog Beispiel 1 mit denselben Molverhältnissen und einer Menge von 14 g Ausgangsstoff II (3-Chlor-o-toluidin) wurden die in Tabelle 2 aufgeführten Umsetzungen mit unterschiedlichen Lösungsmitteln, organischen Säuren und Reaktionstemperaturen durchgeführt. In allen Fällen war die Reinheit des Endstoffs gleich (Fp. 42 bis 43°C). Die Menge an Säure betrug stets 0,5 g, die Menge an Lösungsmittel betrug stets 200 Milliliter.

TABELLE 2

| Nr. | Säure | Temperatur in °C | Lösungsmittel | Ausbeute an Endstoff I in % der Theorie |
|---|---|---|---|---|
| 43 | p-Toluolsulfonsäure | 180 | Dichlorbenzol | 87 |
| 44 | p-Toluolsulfonsäure | 160 | Dimethylsulfoxid | 69 |
| 45 | p-Toluolsulfonsäure | 150 | Dimethylformamid | 73 |
| 46 | p-Toluolsulfonsäure | 160 | Diethylenglykoldiethylether | 78 |
| 47 | Dodecylbenzolsulfonsäure | 180 | Decalin | 82 |
| 48 | p-Toluolsulfonsäure | 130 | Methoxypropanol | 62 |
| 49 | Essigsäure | 110 | Toluol | 74 |
| 50 | Pikrinsäure | 130 | Chlorbenzol | 65 |
| 51 | Propionsäure | 130 | Chlorbenzol | 79 |
| 52 | p-Toluolsulfonsäure | 138 | Xylol | 86 |
| 53 | p-Toluolsulfonsäure | 130 | Methylglykol | 68 |
| 54 | p-Toluolsulfonsäure | 120 | Diethylglykol | 67 |
| 55 | p-Toluolsulfonsäure | 145 | Sulfolan | 75 |

*Beispiel 56*

14 g 3-Chlor-o-toluidin und 0,5 g p-Toluolsulfonsäure wurden in 100 ml Xylol auf 138°C erhitzt. Innerhalb 1 Stunde wurden 26 g Acroleindiethylacetal (gelöst in 50 ml Xylol) in die Lösung eingetragen. Das Gemisch wurde 0,5 Stunden bei obiger Temperatur nachgerührt, anschliessend das Xylol abdestilliert und der Rückstand über eine Kolonne destilliert. Es wurden 12,9 g 7-Chlor-8-methylchinolin (73% der Theorie ) vom Fp. 42 bis 43°C erhalten.

**Patentansprüche**

1. Verfahren zur Herstellung von Chinolinen der Formel

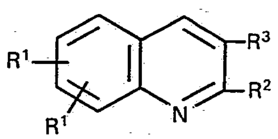

I,

worin die einzelnen Reste $R^1$, $R^2$ und $R^3$ gleich oder verschieden sein können und jeweils ein Wasserstoffatom oder einen aliphatischen Rest bedeuten, die Reste $R^1$ auch jeweils ein Halogenatom, eine Alkoxygruppe, eine Aminogruppe, eine Monoalkyl- oder eine Dialkylaminogruppe, oder zusammen mit zwei benachbarten Kohlenstoffatomen einen Isoxazolrest, Oxazolrest, Thiazolrest, Isothiazolrest, Furanrest, Thiophenrest, Pyrrolrest, Imidazolrest oder Pyrazolrest bezeichnen, durch Umsetzung von aromatischen Aminen der Formel

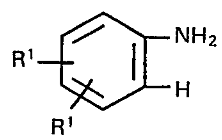

II,

worin $R^1$ die vorgenannte Bedeutung besitzt, mit Acroleinen oder seinen Acetalen der Formel

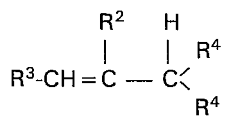

III,

worin die einzelnen Reste $R^4$ gleich oder verschieden sein können und jeweils für eine Alkoxygruppe oder zusammen für ein Sauerstoffatom stehen, in Gegenwart von Säure und organischen Lösungsmitteln, dadurch gekennzeichnet, dass man als Säure organische Säuren verwendet und die Umsetzung in Gegenwart von unter den Reaktionsbedingungen inerten, nitrogruppenfreien, organischen Lösungsmitteln durchführt.

2. Verfahren nach Anspruch 1, dadurch gekennzeichnet, dass man die Umsetzung in Gegenwart katalytischer Mengen an organischer Säure durchführt.

**Claims**

1. A process for the preparation of quinolines of the formula

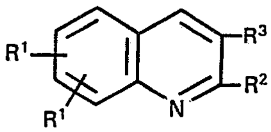

I,

where the individual radicals $R^1$, $R^2$ und $R^3$ may be identical or different and each is hydrogen or an aliphatic radical, the radicals $R^1$ also each denoting halogen or an alkoxy, amino, monoalkyl or dialkylamino group, or, together with two adjacent carbon atoms, an isoxazole, oxazole, thiazole, isothiazole, furan, thiophene, pyrrole, imidazole or pyrazole radical, by reacting aromatic amines of the formula

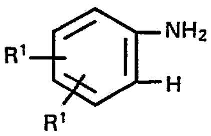

II,

where $R^1$ has the above meanings, with acroleins or their acetals of the formula

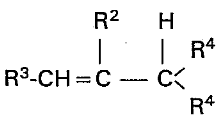

III,

where the individual radicals $R^4$ may be identical or different and are each an alkoxy group or, together, denote oxygen, in the presence of an acid and organic solvents, wherein the acid used is an organic acid, and the reaction is carried out in the presence of organic solvents which are free of nitro groups and inert under the reaction conditions.

2. A process as claimed in claim 1, wherein the reaction is carried out in the presence of a catalytic amount of an organic acid.

**Revendications**

1. Procédé de préparation de quinoléines de la formule

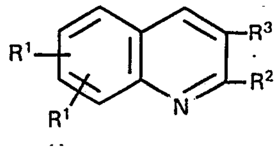

I,

dans laquelle les divers substituants $R^1$, $R^2$ et $R^3$ peuvent être identiques ou différents et désigner chacun un atome d'hydrogène ou un groupe aliphatique, les substituants $R^1$ pouvant en outre désigner chacun un atome d'halogène ou un radical alcoxy ou un groupe amino, monoalkyl-amino ou dialkyl-amino ou former avec deux atomes de carbone voisins un groupe isoxazole, oxazole, thiazole, isothiazole, furanne, thiophène, pyrrole, imidazole ou pyrazole, par la réaction d'amines aromatiques de la formule

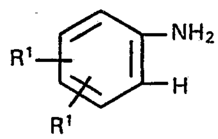

II,

dans laquelle les substituants $R^1$ possèdent la signification définie ci-dessus, dans un solvant organique et en présence d'acide, avec des acroléines ou acétals d'acroléines de la formule

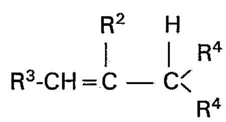

III,

dans laquelle les substituants $R^4$ désignent des radicaux alcoxy identiques ou différents ou représentent ensemble un atome d'oxygène, caractérisé en ce que l'acide est choisi parmi les acides organiques et la réaction est réalisée dans un solvant organique exempt de groupes nitro, inerte dans les conditions opératoires.

2. Procédé suivant la revendication 1, caractérisé en ce que la réaction est effectuée en présence d'une proportion catalytique d'un acide organique.